# EUROPEAN PATENT APPLICATION

(11) **EP 4 293 677 A1**
(43) Date of publication of application: **20.12.2023**
(21) Application number: 22179348.2
(22) Date of filing: 16.06.2022
(51) Int. Cl.: G16H 40/20, G06N 3/08, G06Q 10/10

(54) **DENTAL TREATMENT PROVIDER RECOMMENDATION**

(71) Applicant: Koninklijke Philips N.V., 5656 AG Eindhoven (NL)
(72) Inventor: SPELT, Hanne Adriana Alijda, Eindhoven (NL); KOOIJMAN, Gerben, Eindhoven (NL); JOHNSON, Mark Thomas, Eindhoven (NL)
(74) Representative: Philips Intellectual Property & Standards

(57) **Abstract**

A method and system for generating recommendations for a user (patient) as to a best matching dental treatment provider (i.e. dental practitioner) in accordance with their preferences or needs as to pain, or other characteristics. The method may employ two algorithms: one to predict a measure of pain associated with different treatment styles and based on type of treatment needed, and one to perform two tasks: to relate the patient preferences and/or requirements to the output predictions of the prediction algorithm to thereby determine a recommended treatment style; and to consult a database of real-world treatment providers and find one or more who match the recommended treatment style for the required treatment.

## Description

### FIELD OF THE INVENTION

This invention relates to a method for recommending a dental treatment provider.

### BACKGROUND OF THE INVENTION

Anticipated treatment discomfort or pain is a significant barrier to patient compliance with regular consultation and treatment with dental practitioners. Treatment pain can cause a patient to develop dental anxiety. Experience of pain at a previous dental visit has been shown to be correlated with dental anxiety. An estimated 3-16% of adults suffer from dental phobia. Patient studies indicate that patients believe that dental practitioners (DPs) should pay attention to any discomfort (e.g., pain, stress, or anxiety) during treatment and that DPs should take steps to ameliorate this. However, anticipated treatment discomfort is not a major cause of anxiety for the whole patient population. In a study in which the sample was representative of the general population aged 15 years and over, 60% reported having at least one dental experience involving 'strong pain', of which only 5-6% experienced dental treatment in general to be very painful.

Therefore, it would be advantageous for patients to be able to determine and confirm with their dental practitioner what treatment style, philosophy or approach will be used by the dental practitioner. Indeed, this is a something which a DP may normally provide for, in advance of conducting a certain treatment, by assessment of the patient and their particular needs and by discussion with the patient, for example to establish how nervous they are. For example, more experienced DPs can usually carry out a single treatment with multiple different treatment styles or philosophies and match the needs of the patient to the best treatment style.

### SUMMARY OF THE INVENTION

In considering this issue, it has been recognized by the inventors that the best way to validate a dental practitioner's treatment style or philosophy is by reviewing the pain it on average inflicts during a particular treatment. Thus, as will be presented below, embodiments of this invention propose to use a machine-learning algorithm trained on a patient database that is built using data which provides an indication of pain associated with different treatments and different styles for a plurality of historical treatment sessions of a plurality of different dental practitioners. The data could for example be acquired using biosignals during treatment to assess pain during oral care. The trained algorithm can be applied to classify expected treatment pain levels for a given treatment and for different possible treatment styles. It could also provide a prediction of other known treatment characteristics. A database of nearby treatment providers can then be searched using the results from the machine learning prediction algorithm to find actual practitioners who will perform a needed treatment with a style which, using the results of the prediction algorithm, have been found to best match the patient's preferences and needs.

The invention is defined by the claims.

In accordance with an aspect of the invention, there is provided a computer-implemented method for use in recommending a dental treatment provider to a patient. The method comprises running a prediction algorithm. The prediction algorithm comprises a trained machine learning algorithm. The prediction algorithm is configured to:
receive input variables comprising at least: a type of treatment required by the patient, and
generate, for each of a pre-defined set of possible treatment styles for said type of treatment, an output prediction comprising a predicted measure of treatment pain.

The method further comprises accessing a dental treatment provider database recording information indicative of a set of dental treatment providers, treatment types available at each treatment provider and one or more treatment styles available for each treatment type.

The method further comprises running a recommendation algorithm, configured to:
receive, as inputs: the output from the prediction algorithm, and, an indication of one or more patient preferences or requirements with respect to treatment pain;
determine, as output, based on the inputs, a recommended one of the treatment styles for the patient, and
determine, as a further output, one or more recommended treatment providers from the treatment provider database, based on identifying treatment providers offering the required type of treatment with the recommended treatment style.

The method further comprises generating a data output indicative of the one or more recommended treatment providers, and optionally further indicative of the recommended treatment style.

The type of treatment may for example be one of a pre-defined list of treatment types, for example, oral cleaning, oral probing, tooth extraction.

Thus, the method is able to provide recommended dental treatment providers to a patient which are best matched to the patient's preferences and needs with respect to a balance between treatment pain vs one or more other characteristics of the treatment, such as cost or duration. This provides an improved patient experience by providing the best match to the patient's needs and preference. It provides an overall improved health outcome for the patient by providing the patient greater control and knowledge about the character of the treatment they will receive, and this has been shown to improve compliance with treatment provider visits. Another advantage is improved staff experience by reducing occupational stress for the dentist. Another advantage is lower cost of care by reducing chair time. Another potential advantage is reduced network resource usage through enabling automatic scheduling of appointments in accordance with recommendations generated by the method. Due to the pre-matching of patient needs to the treatment provider, there is greater likelihood of patient attendance, reducing non-attendance rate, which thereby improves efficiency. Fewer appointments need to be made and re-made.

By way of example, the treatment styles could include one or more of: slow, careful approach; more rapid, forceful approach; mechanical approach; pharmaceutical approach. Another example is a minimal anesthesia approach in which anesthesia use is minimized. Another approach is a precautionary antibiotics approach in which antibiotics are administered after any dental treatment, on a 'just in case' basis. Another approach is a minimal antibiotics approach in which antibiotics are administered only if essential. Another approach is a fast/flexible approach in which a practitioner, upon performing dental examination will give the option to perform the needed treatment straight away (even if this may be more painful that waiting for an appointment some weeks later for which preparations could be made for anesthesia). Another approach is a precautionary treatment approach in which superficial caries would be treated with mechanical (drilling) treatment. Another approach (opposite to the precautionary treatment approach) would be the minimal treatment approach in which a response to superficial caries would be to prescribe a high fluoride toothpaste, and/or to administer a fluoride rinse, and to schedule monitoring appointments.

In some embodiments, the input variables to the prediction algorithm further include one or more patient oral health characteristics. The prediction algorithm may be pre-trained in this case to generate predicted pain tailored to the particular one or more health characteristics. One illustrative example for instance is whether the patient is prone to gingivitis, in which case, more pain might be predicted. Other examples of oral health characteristics may include, by way of illustration: presence or absence of sensitive gums, presence of an infection, recent intervention history (e.g. extraction, pocket treatment), or jaw problems (e.g. TMJ - pain opening the jaw).

In some embodiments, the inputs to the recommendation algorithm further include one or more patient personality characteristics. The determining the recommended treatment style and/or determining the one or more recommended treatment providers is further based on said one or more patient personality characteristics.

In some embodiments, the determining the one or more recommended treatment providers is further based on a geographical location of the provider relative to a geographical location of the patient's home.

In some embodiments, the prediction algorithm is further adapted to generate, as part of the output prediction for each of the pre-defined set of possible treatment styles for said type of treatment, at least one further predicted characteristic of the treatment. In this case, optionally, the inputs to the recommendation algorithm may further include an indication of one or more patient preferences or requirements with respect to said at least one further characteristics of the treatment.

In some embodiments, the at least one further characteristic of the treatment includes one or more of: a treatment duration, a treatment cost. Other examples of the at least one further characteristic of the treatment may include: anesthetics use characteristics, antibiotics prescribing practices, availability of flexible follow-up treatment,

In some embodiments, the determining the recommended treatment style and/or the one or more recommended treatment providers is further based on an input indicative of patient preferences or requirements as to: treatment cost, and/or a treatment duration.

In some embodiments, the treatment provider database comprises information indicative of
- clinician communication styles available at each treatment provider; and/or
- stress reduction techniques available at each treatment provider;
and wherein the determining the one or more recommended treatment providers is further based on an input indicative of patient preferences regarding communication style and/or stress-reduction techniques.

In some embodiments, the prediction algorithm is a machine learning algorithm pre-trained using a training database comprising respective data entries for each of a plurality of prior/historical visits by a plurality of different patients to a set of different dental treatment providers, and wherein each visit entry includes at least an indication of:
- a type of treatment administered;
- a treatment style, where the treatment style is one of the said pre-defined set of treatment styles; and
- a measure indicative of treatment pain.

The database entries may further each include an indication of said at least one further characteristic of the treatment, which could include for instance treatment duration and/or treatment cost.

It is noted that the treatment providers whose treatment data is included in this training database need not (and typically are not) the same treatment providers as are included in the second 'treatment provider database' which has been introduced above, the latter of which is searched to find actual real-world treatment providers to recommend to the patient. It is anticipated that relatively few, if any, of the treatment providers local to a given patient will have accumulated and contributed the specialist type of treatment data which is present in the training database. This indeed is in part the motivation behind the inventors proposing use of a trained machine learning algorithm: the algorithm can make a prediction about the level of pain associated with different treatment styles, and potentially given other characteristics of the patient, despite there not being specific historical data for the patient's local treatment providers which can be consulted in order to verify this. The prediction algorithm helps to narrow down a treatment style / at least one other treatment characteristic based on the (ideally) large training database; then the (typically smaller) real-world treatment provider database can be consulted to find a treatment provider best matching the patient's needs in view of the output of the prediction algorithm.

In some embodiments, at least a subset of the visit entries further include an indication of:
a treatment duration;
a treatment cost;
one or more oral health characteristics of the patient;
a geographical location of the treatment provider; and/or
one or more patient personality characteristics.

In some embodiments, the measure indicative of treatment pain is a quantitative measure.

In some embodiments, the measure indicative of treatment pain corresponds to a measured total pain amount, the measured total pain amount corresponding to a product of a measured pain response level and a time for which the pain response level was experienced, for each of one or more pain response episodes.

For example, this information could be derived from a biological sensing signal acquired as a function of time during the treatment, e.g. a skin conductance signal or another biological signal. Alternatively, the measure of treatment pain could be a measure from visual analog scales.

Indeed, another aspect of this invention could be the composing of the training database through the acquisition of the treatment session data entries, including the acquisition of the biosignal-based pain quantification data mentioned above. This will be explained with greater detail in the following section of this document.

Another aspect of the invention could be the training of the machine learning algorithm which is comprised by the prediction algorithm, using the training database. This also will be explained with greater particularity in the following section of this document.

In some embodiments, the method may comprise controlling a user interface to generate a user-perceptible indication of the one or more recommended treatment providers, and/or the recommended treatment style.

In some embodiments, the method may comprise receiving a user-input from a user interface indicative of a user selection or confirmation of one of the one or more recommended treatment providers.

In some embodiments, the method may further comprise a preliminary step of:
accessing a further treatment provider database recording information indicative of a set of dental treatment providers, treatment types available at each treatment provider, one or more treatment styles available for each treatment type, and a measure of treatment pain associated with each treatment type and each treatment style; and
determining whether the further treatment provider database includes one or more treatment providers within a threshold geographical proximity of the patient and, if so, using the further treatment provider database instead of the prediction algorithm to determine, for each of a pre-defined set of possible treatment styles for said type of treatment, an output comprising a predicted measure of treatment pain.

In some embodiments, the further treatment provider database is the same database which is used to train the machine learning algorithm of the prediction algorithm, i.e. the training database referred to previously.

In some embodiments, the treatment provider database, further treatment provider database and the training database are all the same identical database.

In some embodiments, the method may comprise generating a consultation scheduling request message containing patient identification information corresponding to the patient.

In some embodiments, the method may comprise transmitting the consultation scheduling request message via a communication channel to (e.g. a computing device or server or other electronic communication address of) the one selected or confirmed treatment provider.

In some embodiments, the consultation scheduling request message further includes information indicative of at least one preferred timing for a consultation with the treatment provider, and preferably also information indicative of a preferred treatment style.

For example, the above scheduling request steps could be performed by a computing device of the patient, for example a mobile computing device such as a smartphone, with the communication channel being an Internet communication channel.

In some embodiments, the method further comprises receiving a scheduling response message from the treatment provider to which the request message was transmitted.

In some embodiments, the response is indicative of an appointment scheduling acceptance, accepting one of the at least one preferred timings included in the request message; or an appointment scheduling rejection, rejecting all of the preferred timings included in the request message, and optionally further including an alternative timing suggestion.

In some embodiments, the method further comprises accessing an electronically stored patient agenda or calendar, and updating the agenda or calendar based on the response message.

Again, the above steps could be performed by a computing device of the patient.

In some embodiments, the method comprises a scheduling system of the treatment provider receiving the consultation scheduling request message.

In some embodiments, the method comprises accessing an electronically stored treatment provider agenda or calendar.

In some embodiments, the method comprises, responsive to determining that one of the at least one preferred timings corresponds to an available consultation timing within the treatment provider agenda or calendar, automatically updating the treatment provider agenda or calendar with a new scheduled consultation with the patient, and generating a response message indicative of an appointment scheduling acceptance of the said one of the at least one preferred timings.

In some embodiments, the method comprises, responsive to determining that none of the at least one preferred timings corresponds to an available consultation timing within the treatment provider agenda or calendar, generating a response message indicative of an appointment scheduling rejection.

In some embodiments, the method comprises transmitting the generated response message to the patient.

The inventive concept can also be embodied in the form of a computer program product with computer program code means configured, when run on a processor, to cause the processor to perform a method in accordance with any embodiment or example described in this disclosure, or in accordance with any claim of this patent application.

The inventive concept can also be embodied in hardware form. Thus, another aspect of the invention is a processing unit for use in recommending a dental treatment provider to a patient, the processing unit comprising one or more processors: The processing unit is configured to run a prediction algorithm, the prediction algorithm comprising a trained machine learning algorithm, and the prediction algorithm configured to:
receive input variables comprising at least: a type of treatment required by the patient, and
generate, for each of a pre-defined set of possible treatment styles for said type of treatment, an output prediction comprising a predicted measure of treatment pain.

The processing unit is further configured to access a dental treatment provider database recording information indicative of a set of dental treatment providers, treatment types available at each treatment provider and treatment styles available for each treatment type.

The processing unit is further configured to run a recommendation algorithm, configured to:
receive, as inputs: the output from the prediction algorithm, and, an indication of one or more patient preferences or requirements with respect to treatment pain;
determine, as output, based on the inputs, a recommended one of the treatment styles for the patient, and
determine, as a further output, one or more recommended treatment providers from the treatment provider database, based on identifying treatment providers offering the required type of treatment with the recommended treatment style.

The processing unit is further configured to generate a data output indicative of the one or more recommended treatment providers, and optionally further indicative of the recommended treatment style.

The processing unit may additionally comprise an input/output and wherein the data output is coupled to the input/output for export from the processing unit, and for example wherein the accessing the database is performed via transfer of data signals through the input/output. The various inputs to the prediction algorithm and/or the recommendation algorithm may also be received at the input/output.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 outlines steps of an example method in accordance with one or more embodiments of the invention;
Fig. 2 schematically outlines components and a processing flow of an example processing arrangement in accordance with one or more embodiments of the invention;
Fig. 3 schematically illustrates processing flow in accordance with one or more embodiments of the invention; and
Fig. 4 schematically outlines an example system facilitating consultation scheduling in accordance with one or more embodiments of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method and system for generating recommendations for a user (patient) as to a best matching dental treatment provider (i.e. dental practitioner) in accordance with their preferences or needs as to pain, or other characteristics. The other characteristics could for example include a preference as to cost or duration of the treatment. The other characteristics could also include patient oral health characteristics and/or personality characteristics. The method may employ two algorithms: one to predict a measure of pain and optionally other treatment characteristics associated with different treatment styles, and one to perform two tasks: to relate the patient preferences and/or requirements to the output predictions of the prediction algorithm to thereby determine a recommended treatment style; and to consult a database of real-world treatment providers and find one or more who match the recommended treatment style for the required treatment, and optionally also which best match one or more other patient preferences or needs, such as with regards to cost, duration, available stress reduction techniques, communication styles etc.

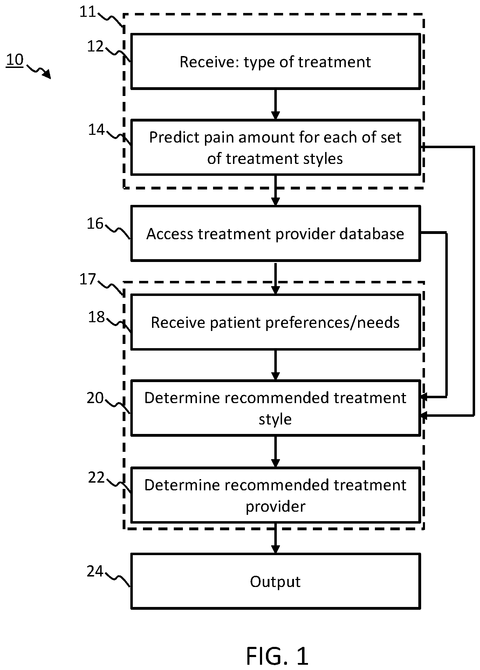

Fig. 1 outlines in block diagram form steps of an example method according to one or more embodiments. The steps will be recited in summary, before being explained further in the form of example embodiments.

The method 10 comprises running a prediction algorithm (schematically indicated by box 11) which comprises a trained machine learning algorithm. The prediction algorithm is configured to receive 12 input variables comprising at least: a type of treatment required by the patient; and to generate 14, for each of a pre-defined set of possible treatment styles for said type of treatment, an output prediction comprising a predicted measure of treatment pain, and optionally at least one further predicted characteristic of the treatment.

The method 10 further comprises accessing 16 a dental treatment provider database recording information indicative of a set of dental treatment providers, treatment types available at each treatment provider and treatment styles available for each treatment type.

The method 10 further comprises running a recommendation algorithm (schematically indicated by box 17), configured to receive, as inputs: the output from the prediction algorithm, and, an indication 18 of one or more patient preferences or requirements with respect to treatment pain and optionally said at least one further characteristic of the treatment. The recommendation algorithm 17 is further configured to determine 20, as output, based on the inputs, a recommended one of the treatment styles for the patient. The recommendation algorithm 17 is further configured to determine 22, as a further output, one or more recommended treatment providers from the treatment provider database, based on identifying treatment providers offering the required type of treatment with the recommended treatment style.

The method 10 further comprises generating 24 a data output indicative of the one or more recommended treatment providers, and optionally further indicative of the recommended treatment style.

Although in the description above, the various steps of the method are designated as being performed by specific algorithms, it will be appreciated that in other examples, the steps of the method may all be performed by a single algorithm, or by more than two algorithms. The inventive concept is not limited according to any particular division of steps between particular algorithms. The designation is for convenience of description and reflects a most likely implementation in practice. Purely by way of one illustration, the designation of steps recited above to the recommendation algorithm could be broken down further and performed by two separate algorithms, for example one to determine the recommended treatment style and another to determine the recommended treatment provider.

By way of example, the treatment styles could include one or more of: slow, careful approach; more rapid, forceful approach; mechanical approach; pharmaceutical approach. Another example is a minimal anesthesia approach in which anesthesia use is minimized. Another approach is a precautionary antibiotics approach in which antibiotics are administered after any dental treatment, on a 'just in case' basis. Another approach is a minimal antibiotics approach in which antibiotics are administered only if essential. Another approach is a fast/flexible approach in which a practitioner, upon performing dental examination will give the option to perform the needed treatment straight away (even if this may be more painful that waiting for an appointment some weeks later for which preparations could be made for anesthesia). Another approach is a precautionary treatment approach in which superficial caries would be treated with mechanical (drilling) treatment. Another approach (opposite to the precautionary treatment approach) would be the minimal treatment approach in which a response to superficial caries would be to prescribe a high fluoride toothpaste, and/or to administer a fluoride rinse, and to schedule monitoring appointments.

With respect to the method of this invention, the pre-defined set of treatment styles can be defined in whatever way is desired. Each would be given a label or name, and a definition of it (in terms of its characteristics) would be defined in advance. This would be known to all of the treatment providers listed in the treatment provider database. Then, when compiling (in advance of the method) the treatment provider database, each treatment provider can review the list of defined treatment styles and the definition of each, and decide which style(s) they currently operate with or are able to operate with. Likewise, when compiling the training database used to train the prediction machine learning algorithm, the treatment providers who contribute data to this database would also be provided the list of pre-defined treatment styles with its definitions of each style, and in this way they could label or tag each treatment session data entry with the corresponding style that best matches the style that was used for that treatment.

Following the above, there is consistency of definition across all parts of the method and system, including all databases and algorithms, in terms of the definitions of the treatment styles.

As noted above, the method can also be embodied in hardware form, for example in the form of a processing unit which is configured to carry out a method in accordance with any example or embodiment described in this document, or in accordance with any claim of this application.

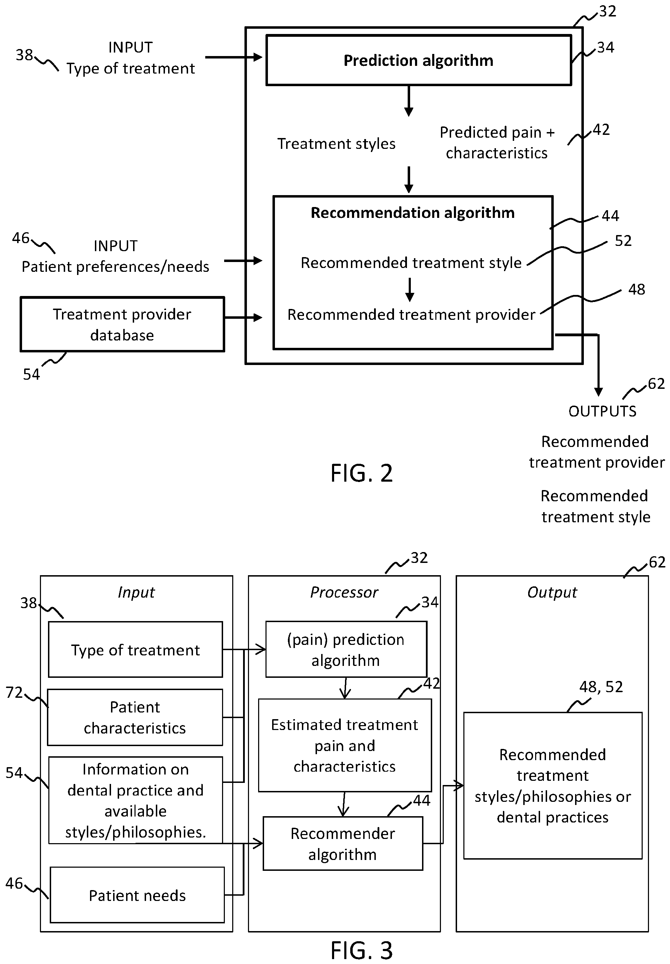

To further aid understanding, Fig. 2 presents a schematic representation of an example processing unit 32 configured to execute a method in accordance with one or more embodiments of the invention, and also schematically showing the processing flow in more detail. The processing unit comprises one or more processors (not shown) and may comprise an input/output. The one or more processors are adapted to run a prediction algorithm 34, the prediction algorithm comprising a trained machine learning algorithm, and the prediction algorithm configured to
receive input variables 38 comprising at least: a type of treatment required by the patient, and
generate, for each of a pre-defined set of possible treatment styles for said type of treatment, an output prediction 42 comprising a predicted measure of treatment pain, and optionally at least one further predicted characteristic of the treatment.

The processing unit 32 is further adapted to access a dental treatment provider database 54 recording information indicative of a set of dental treatment providers, treatment types available at each treatment provider and treatment styles available for each treatment type.

The processing unit 32 is further adapted to run a recommendation algorithm 44, configured to:
receive, as inputs: the output 42 from the prediction algorithm, and, an indication 46 of one or more patient preferences or requirements with respect to treatment pain and optionally said at least one further characteristic of the treatment;
determine, as output, based on the inputs, a recommended one 52 of the treatment styles for the patient, and
determine, as a further output, one or more recommended treatment providers 48 from the treatment provider database, based on identifying treatment providers offering the required type of treatment with the recommended treatment style.

The processing unit is further adapted to generate a data output 62 indicative of the one or more recommended treatment providers, and optionally further indicative of the recommended treatment style.

By way of illustration, an example implementation of the method according to a particular set of embodiments considered particularly advantageous by the inventors, could be summarized as comprising the following features:
a processor to execute one or more algorithms that:
   receives information on the patient's needs and optionally patient's characteristics;
   receives information on requirements for an upcoming consultation of the patient, i.e. the type of treatment needed;
   has access to a database with information about nearby dental practices and their treatment styles/philosophies;
predicts treatment characteristics (e.g., expected pain, time, cost) for the upcoming treatment;
recommends a best dental practice and/or treatment philosophy based on patient needs and the database.

This processor could be part of a computing device of the patient, for example a mobile computing device, for example a smartphone or tablet. This could be part of a dental device in other examples. It could be part of a cloud-based server in some examples. It could be part of a computing workstation in some examples.

As noted above, a part of the method is the use of a prediction algorithm in the form of a trained machine learning algorithm. Details in respect of the trained machine learning algorithm will now be discussed in further detail.

A machine-learning algorithm is any self-training algorithm that processes input data in order to produce or predict output data. In the present case, the input data comprises, at minimum, the treatment type required by the patient, and the output data comprises a predicted measure of treatment pain for each of a pre-defined set of treatment styles, and optionally one or more further predicted characteristics of the treatment.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines, Naive Bayesian models, and k-nearest neighbor models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

In the present case, the training dataset may be based on data contained in a database which comprising respective data entries for each of a plurality of prior/historical treatment sessions by a plurality of different patients to a set of different dental treatment providers, and wherein each treatment session entry includes at least an indication of:
- a type of treatment administered;
- a treatment style, where the treatment style is one of the said pre-defined set of treatment styles; and
- a measure indicative of treatment pain.

In this case, the training input data entries of the training data may correspond to the type of treatment needed by the patient and the treatment style. The training output data entries may correspond to a measure indicative of predicted pain. This way, when running the trained prediction algorithm in the context of the present invention, to generate the list of predicted measures of treatment pain for each of the set of different possible treatment styles, the algorithm could be run a plurality of times, one time for each of the different possible treatment styles, thereby resulting in an output prediction dataset which gives the necessary pain prediction for each of the different treatment styles.

Another alternative would be to use a prediction algorithm which comprises a plurality of trained machine learning algorithms, wherein each has been trained using a filtered subset of the training dataset comprising only those training data entries which correspond to a particular respective one of the treatment styles. In this case, the training input data for each of the machine learning algorithms, may comprise just the type of treatment (at minimum) and the training output data entries may comprise (at minimum) just the predicted measure of treatment pain. Then, when running the prediction algorithm in the context of the present invention, each of the trained machine learning algorithms comprised by the overall prediction algorithm may be run with an input comprising the type of treatment needed by the patient, and each generates an output indicative of a predicted measure of pain for the particular treatment style for which it has been trained to predict.

In some embodiments, at least a subset of the treatment session data entries in the training database may additionally include an indication of one or more of: a treatment duration; a treatment cost; one or more oral health characteristics of the patient; and/or a geographical location of the treatment provider; and wherein the machine learning algorithm is trained accordingly.

In some embodiments, the measure indicative of treatment pain included in the data entries is a quantitative measure. For example, it may correspond to a measured total pain amount. By way of example, the total measured pain amount may correspond to a product of a measured pain response level and a time for which the pain response level was experienced, for each of one or more pain response episodes.

For example, the pain information can be derived from a biological sensing signal acquired as a function of time during the treatment in the plurality of historical treatment sessions.

By way of one example, during a treatment session, an objective measure of treatment pain can be derived in the following way, for generating the data entry for the database.

A processor at the treatment provider office can be adapted for classifying a subject's response to a series of contact events during a measurement session, the processor adapted to: receive, during the measurement session, an indication of timings of a series of oral contact events, each contact event corresponding to mechanical engagement of an oral tool with a surface within the oral cavity of a subject; receive, during the measurement session, a biological signal for the subject from a biological signal sensing apparatus coupled to the subject; identify physiological response events in the biological signal; determine a temporal proximity between each physiological response event and an immediately preceding contact event, and classify a subject pain and/or non-pain arousal associated with the physiological response event based thereon. For example, the processor is configured to classify a physiological response event as associated with a pain state dependent on the physiological response event occurring within a pre-defined time window immediately following a contact event. The classification can further comprise determining a pain level of the pain state based on one or more properties of the physiological response event peak or drop, and preferably wherein the one or more properties include a maximum height of a leading edge of the peak or drop. The classification can be performed in real time during the measurement session.

The processor can be adapted to compute a total pain amount over the measurement session, the total pain amount being a product of a pain level and a time for which the pain was experienced, the pain level being computed for each physiological response event peak or drop having a pain classification based on one or more properties of the physiological response event peak or drop. The processor can also be configured to classify a physiological response event as associated with a non-pain arousal state dependent on the physiological response event occurring outside of a time window of pre-defined duration following any contact event. The processor could optionally be adapted to further compute a total non-pain arousal amount over the measurement session, the total non-pain arousal amount being a product of an arousal level and a time for which the arousal was experienced, the arousal level being computed for each physiological response event peak or drop having a non-pain arousal classification based on one or more properties of the physiological response event peak or drop.

Within the office of the treatment provider, there could be a system comprising: a contact identification means for identifying timings of a series of oral contact events, each contact event corresponding to mechanical engagement of an oral tool with a surface within the oral cavity of a subject; a biological signal sensing apparatus for measuring a physiological response of the subject (such as a skin conductance sensor or heart rate sensor or any other biosignal sensor); and a processor as already described, arranged to receive, over a measurement session, one or more contact signals from the contact identification means indicative of timings of the series of oral contact events and a biological signal from the biological signal sensing apparatus.

Thus, it can be recognized how an objective measure of a pain associated with a particular treatment can be acquired based on biosignals. The measure of treatment pain recorded in the database used to train the machine learning algorithm could, for each database treatment session entry, correspond to the total pain amount for the treatment session, or could correspond to the highest experienced pain level during each session, or could correspond to an average pain level throughout the session, or ratio of total pain to total non pain amount, or any other variable or parameter derived from the biosignal pain data discussed above acquired during each historical treatment session.

This information could then be stored as an entry in the database which is used to train the prediction algorithm 34, along with indication of the type of treatment administered, and a treatment style, where the treatment style is one of the said pre-defined set of treatment styles.

Indeed, another aspect of this invention may be the compilation of the training dataset referred to above, using the pain measurement technique mentioned above, or another objective pain measurement technique.

Another aspect of the invention may be the training of the prediction algorithm in accordance with the example method already described above.

An aspect of the invention could include a combination of the compilation of the training database using the pain measurement technique described above and the training of the prediction algorithm using this training database in accordance with the example method described above.

An embodiment of the invention may comprise:
the compilation of the training database using for example one or more features of the pain measurement technique described above, or another technique;
the training of the machine learning algorithm comprised by the prediction algorithm using this training database; and
the execution of the treatment provider recommendation method steps already recited above with reference to Fig. 1 (or any other embodiment / example of this method outlined in this document, or in accordance with any claim of this application).

An example implementation of a method according to a particular set of embodiments of the invention will now be described by way of illustration of the concept of the invention summarized in Fig. 1. It will be appreciated that not all features of this particular set of embodiments are essential to the inventive concept, and are described to aid understanding and to provide an example to illustrate the inventive concepts.

In accordance with this particular set of embodiments, a system and method is provided which recommends the best dental practice and/or treatment style or philosophy to a patient, considering their needs and characteristics. To predict the expected treatment pain and other treatment characteristics, a machine learning algorithm is trained using a large patient database. The database stores results from pain measurement data recorded using for example an objective pain monitoring apparatus such as that outlined already above. The database includes for each case at least information on:
type of treatment (e.g. cleaning);
style or philosophy of treatment (e.g. mechanical, pharmaceutical, slow careful approach, rapid forceful approach);
experienced treatment pain (e.g. based on a visual analogue scale metric, or based on biosignal-based measurements);
and optionally, one or more of: treatment duration; treatment cost; one or more patient characteristics (e.g., prone to gingivitis).

The processing flow according to this particular set of embodiments is outlined schematically in Fig. 3. The prediction algorithm 34 receives information on the type of treatment 38 required by the patient (e.g., cleaning, probing, extraction), and optionally one or more of the patient's oral health characteristics 72 (e.g., sensitive gums). This information is gathered for example by an online survey, conducted by the patient themselves, or a caregiver. For example, the patient may log in to an online portal which provides a graphical user interface with various fields which prompt for relevant information.

As mentioned above, the machine learning algorithm of the prediction algorithm 34 can be previously trained on a database that includes treatment characteristics of dental practices, the treatment styles and experienced treatment pain for each given treatment (and style).

The same or another algorithm further has access to a reduced database 54 with information on dental practices geographically close to the patient and the treatments and treatment styles available there.

Based on the inputs 38, 72 and the reduced database 54, the prediction algorithm 34 predicts the expected treatment pain and optionally other treatment characteristics of the potential upcoming treatment for this patient (e.g., time duration, cost, dose of anesthesia).

The same or a different algorithm 44 receives as input an indication of the needs or preferences 46 of the patient with respect to the upcoming treatment (e.g., cheap, fast, careful). Needs could be practical (e.g., fast, cheap), or emotional (e.g., extra explanation desired, relaxation techniques desired, ability to bring a friend, etc.). Then the algorithm 44 produces a list 48, 52 of potential dental practices and treatment styles/philosophies, preferably ordered by best match considering the patient needs and the estimated treatment characteristics. This list is then fed back to the patient to choose from, for example on a user interface of a computing device such as smartphone. Optionally, the list may be to benchmark the treatment philosophy and skill of different staff members within one dental practice or between dental practices - thereby improving the quality of care.

According to an extension of the above-described example set of embodiments, a further input to the recommendation algorithm may be an indication of one or more personality characteristics of the patient, so that the recommended treatment style and/or recommended treatment provider(s) may take account of personality characteristics. It has been shown that personality characteristics can play a role in the development of dental anxiety. In particular, anxiety correlates positively with neuroticism and symptoms of depression and correlates negatively with extraversion (the reader is referred to the document: Halonen, H. (2021). Dental anxiety: a patient's personality traits and comorbidity with other psychiatric disorders (Doctoral dissertation, Väitöskirja. Oulun yliopisto)).

Personality characteristics also play a role in the effectivity of dental anxiety reduction techniques. For example, people with a greater need for cognition are likely more at ease when a dental practitioner verbally explains what they are doing during treatment. Dental practitioners might have their own preferred methods to put patients at ease during a consultation. They may provide extra explanation, reassuring words, the opportunity to do paced breathing or provide stress reduction modules such as through virtual reality (VR) guidance. In this set of embodiments the patient characteristics 72 can thus also include an indication of one or more relevant personality characteristics (such as Big 5 personality, Need for Cognition, self-consciousness). The presented list of recommended treatment providers may additionally include an overview of available stress reduction methods at each treatment provider. In other words, the inputs to the recommendation algorithm 44 further include one or more patient personality characteristics, and the determining the recommended treatment style and/or determining the one or more recommended treatment providers is further based on said one or more patient personality characteristics.

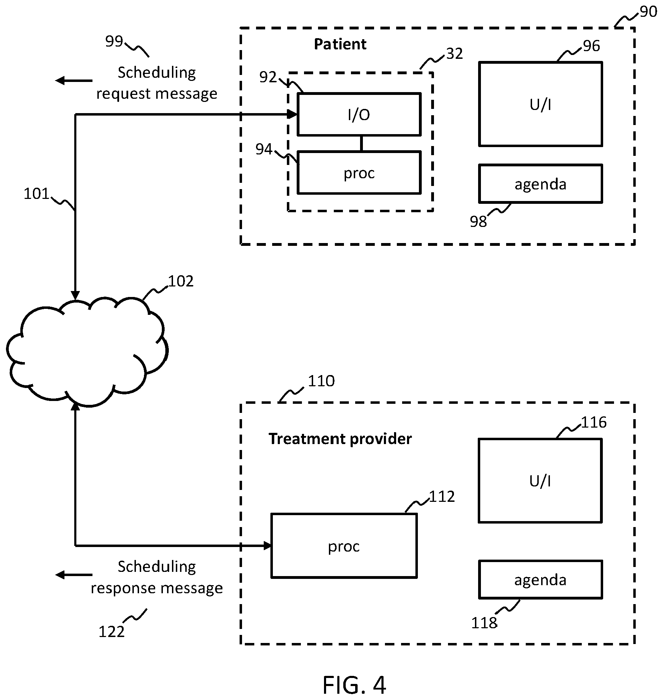

According to some embodiments, means is provided for facilitating communication between a processing unit which generates the recommendations as to treatment provider, and the recommended treatment provider for scheduling purposes or other purposes. To illustrate, Fig. 4 schematically illustrates an example system in which this concept is embodied. Illustrated is an example computing device 90, for example a mobile computing device such as a smartphone or tablet computer, belonging to the patient. The processing unit 32 already discussed is shown. The processing unit comprises one or more processors 94 and an input/output 92. The patient computing device further comprises a user interface 96, for example a display unit such as a touchscreen display. The user interface should facilitate both output of information to the user and input of information by the user. The user interface for example can be used to generate a user-perceptible indication of the one or more recommended treatment providers. The user interface can also be used in some examples to obtain the input information indicative of type of treatment 38 needed and/or patient characteristics 72, and/or patient preferences or needs 46 as to pain and/or other characteristics of the treatment. It is noted that although the patient computing device is shown as being the device which houses the processor 32 which executes the method of the invention, in fact, instead, the computing device could be used primarily as a data input/output terminal, and wherein all or most of the steps of the main method are performed by a remote processor, for example a cloud-based processor.

In accordance with at least one set of embodiments, the user interface 96 is controlled to generate a user-perceptible indication of the one or more recommended treatment providers. The user interface 96 may further be used to receive an indication of a user selection or confirmation of one of the one or more recommended treatment providers provided at the user interface. The processing unit 32 may further be configured to automatically generate a consultation scheduling request message 99 containing patient identification information corresponding to the patient, and to transmit the consultation scheduling request message via a communication channel 101 to a computing device 110 of the one selected or confirmed treatment provider. The communication channel may for example be an Internet communication channel. It may be mediated by a dedicated server 102, for example a cloud-based server, which facilitates communication between patient users and treatment provider users. The scheduling request message may be generated by the processing unit 32 at the patient end after accessing and consulting an electronic agenda or calendar 98 of the patient, or based on a patient inputting their preference as to timings.

The consultation scheduling request message 99 preferably further includes information indicative of at least one preferred timing for a consultation with the treatment provider.

At the computing device 110 of the treatment provider, the scheduling request message 99 is received. The computing device of the treatment provider may form part of or represent a scheduling system of the treatment provider. Upon receipt of the message at a processor 112 of the computing device 110, the processor 112 accesses an electronically stored treatment provider agenda or calendar 118 and checks whether any one of the at least one preferred timings corresponds to an available consultation timing within the treatment provider agenda or calendar.

Responsive to determining that one of the at least one preferred timings corresponds to an available consultation timing within the treatment provider agenda or calendar 118, the processor 112 automatically updates the treatment provider agenda or calendar with a new scheduled consultation with the patient, and generates a response message 122 indicative of an appointment scheduling acceptance of the said one of the at least one preferred timings. In the alternative, responsive to determining that none of the at least one preferred timings corresponds to an available consultation timing within the treatment provider agenda or calendar, the processor 112 generates a response message 122 indicative of an appointment scheduling rejection, and optionally further including an alternative timing suggestion. The generated response message is transmitted to the patient computing device 90.

At the patient device 90 end, the processing unit 32 receives the scheduling response message 122 from the treatment provider, the response indicative of either the appointment scheduling acceptance, accepting one of the at least one preferred timings included in the request message 99, or an appointment scheduling rejection, rejecting all of the preferred timings included in the request message, and optionally further including an alternative timing suggestion. The processing unit 32 further accesses the electronically stored patient agenda or calendar 98, and updates the agenda or calendar based on the response message.

As mentioned previously, the invention can be embodied in software form. Thus another aspect of the invention is a computer program product comprising code means configured, when run on a processor, to cause the processor to perform a method in accordance with example or embodiment of the invention described in this document, or in accordance with any claim of this patent application.

Embodiments of the invention described above employ a processing unit. The processing unit may in general comprise a single processor or a plurality of processors. It may be located in a single containing device, structure or unit, or it may be distributed between a plurality of different devices, structures or units. Reference therefore to the processing unit being adapted or configured to perform a particular step or task may correspond to that step or task being performed by any one or more of a plurality of processing components, either alone or in combination. The skilled person will understand how such a distributed processing arrangement can be implemented. The processing unit may include a communication module or input/output for receiving data and outputting data to further components.

The one or more processors of the processing unit can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A computer-implemented method for use in recommending a dental treatment provider to a patient, the method comprising:
running a prediction algorithm, the prediction algorithm comprising a trained machine learning algorithm, and the prediction algorithm configured to
receive input variables comprising at least: a type of treatment required by the patient, and
generate, for each of a pre-defined set of possible treatment styles for said type of treatment, an output prediction comprising a predicted measure of treatment pain;
accessing a dental treatment provider database recording information indicative of a set of dental treatment providers, treatment types available at each treatment provider and one or more treatment styles available for each treatment type;
running a recommendation algorithm, configured to:
receive, as inputs: the output from the prediction algorithm, and, an indication of one or more patient preferences or requirements with respect to treatment pain;
determine, as output, based on the inputs, a recommended one of the treatment styles for the patient, and
determine, as a further output, one or more recommended treatment providers from the treatment provider database, based on identifying treatment providers offering the required type of treatment with the recommended treatment style;
generating a data output indicative of the one or more recommended treatment providers, and optionally further indicative of the recommended treatment style.

2. The method of claim 1, wherein the input variables to the prediction algorithm further include one or more patient oral health characteristics.

3. The method of claim 1 or 2, wherein
the inputs to the recommendation algorithm further include one or more patient personality characteristics, and
determining the recommended treatment style and/or determining the one or more recommended treatment providers is further based on said one or more patient personality characteristics.

4. The method of any of any of claims 1-3, wherein the prediction algorithm is further adapted to generate, as part of the output prediction for each of the pre-defined set of possible treatment styles for said type of treatment, at least one further predicted characteristic of the treatment, and
optionally wherein the at least one further characteristic of the treatment includes one or more of: a treatment duration, a treatment cost.

5. The method of claim 4, wherein determining the recommended treatment style and/or the one or more recommended treatment providers is further based on an input indicative of patient preferences or requirements as to: treatment cost, and/or a treatment duration.

6. The method of any of claims 1-5, wherein the treatment provider database comprises information indicative of
- clinician communication styles available at each treatment provider; and/or
- stress reduction techniques available at each treatment provider;
and wherein the determining the one or more recommended treatment providers is further based on an input indicative of patient preferences regarding communication style and/or stress-reduction techniques.

7. The method of any of claims 1-6, wherein the prediction algorithm is a machine learning algorithm pre-trained using a training database comprising respective data entries for each of a plurality of prior visits by a plurality of different patients to a set of different dental treatment providers, and wherein each visit entry includes at least an indication of:
- a type of treatment administered;
- a treatment style, where the treatment style is one of the said pre-defined set of treatment styles; and
- a measure indicative of treatment pain.

8. The method of claim 7, wherein at least a subset of the visit entries further include an indication of:
a treatment duration;
a treatment cost;
one or more oral health characteristics of the patient;
a geographical location of the treatment provider; and/or
one or more patient personality characteristics.

9. The method of claim 7 or 8,
wherein the measure indicative of treatment pain is a quantitative measure; and
optionally wherein the measure indicative of treatment pain corresponds to a measured total pain amount, the measured total pain amount corresponding to a product of a measured pain response level and a time for which the pain response level was experienced, for each of one or more pain response episodes.

10. The method of any of claims 1-9, further comprising:
controlling a user interface to generate a user-perceptible indication of the one or more recommended treatment providers;
receiving a user-input from the user interface indicative of a user selection or confirmation of one of the one or more recommended treatment providers;
generating a consultation scheduling request message containing patient identification information corresponding to the patient; and
transmitting the consultation scheduling request message via a communication channel to the one selected or confirmed treatment provider.

11. The method of claim 10, wherein the consultation scheduling request message further includes information indicative of at least one preferred timing for a consultation with the treatment provider, and preferably information indicative of a preferred treatment style.

12. The method of claim 11, wherein the method further comprises:
receiving a scheduling response message from the treatment provider to which the request message was transmitted, the response indicative of:
an appointment scheduling acceptance, accepting one of the at least one preferred timings included in the request message; or
an appointment scheduling rejection, rejecting all of the preferred timings included in the request message, and optionally further including an alternative timing suggestion; and
accessing an electronically stored patient agenda or calendar, and updating the agenda or calendar based on the response message.

13. The method of any of claims 11-12, further comprising:
a scheduling system of the treatment provider receiving the consultation scheduling request message;
accessing an electronically stored treatment provider agenda or calendar;
responsive to determining that one of the at least one preferred timings corresponds to an available consultation timing within the treatment provider agenda or calendar, automatically updating the treatment provider agenda or calendar with a new scheduled consultation with the patient, and generating a response message indicative of an appointment scheduling acceptance of the said one of the at least one preferred timings;
responsive to determining that none of the at least one preferred timings corresponds to an available consultation timing within the treatment provider agenda or calendar, generating a response message indicative of an appointment scheduling rejection;
transmitting the generated response message to the patient.

14. A computer program product comprising code means configured, when run on a processor, to cause the processor to perform a method in accordance with any of claims 1-13.

15. A processing unit for use in recommending a dental treatment provider to a patient, the processing unit comprising one or more processors and configured to:
run a prediction algorithm, the prediction algorithm comprising a trained machine learning algorithm, and the prediction algorithm configured to
receive input variables comprising at least: a type of treatment required by the patient, and
generate, for each of a pre-defined set of possible treatment styles for said type of treatment, an output prediction comprising a predicted measure of treatment pain;
access a dental treatment provider database recording information indicative of a set of dental treatment providers, treatment types available at each treatment provider and one or more treatment styles available for each treatment type;
run a recommendation algorithm, configured to:
receive, as inputs: the output from the prediction algorithm, and, an indication of one or more patient preferences or requirements with respect to treatment pain;
determine, as output, based on the inputs, a recommended one of the treatment styles for the patient, and
determine, as a further output, one or more recommended treatment providers from the treatment provider database, based on identifying treatment providers offering the required type of treatment with the recommended treatment style;
generate a data output indicative of the one or more recommended treatment providers, and optionally further indicative of the recommended treatment style.
